(19) Europäisches Patentamt
European Patent Office
Office européen des brevets

(11) Numéro de publication : **0 045 234**
**B1**

(12) **FASCICULE DE BREVET EUROPÉEN**

(45) Date de publication du fascicule du brevet : **23.05.84**

(21) Numéro de dépôt : **81401098.9**

(22) Date de dépôt : **08.07.81**

(51) Int. Cl.³ : **C 07 C 85/20**, C 07 D307/93, C 07 D307/91, C 07 D451/02, C 07 D295/02

(54) **Nouveau procédé de déalkylation des amines tertiaires par emploi de chloroformiates alpha-chlorés.**

(30) Priorité : **24.07.80 FR 8016311**

(43) Date de publication de la demande : **03.02.82 Bulletin 82/05**

(45) Mention de la délivrance du brevet : **23.05.84 Bulletin 84/21**

(84) Etats contractants désignés : **AT BE CH DE FR GB IT LI LU NL SE**

(56) Documents cités : **US-A- 3 905 981**

(73) Titulaire : **SOCIETE NATIONALE DES POUDRES ET EXPLOSIFS**
**12, quai Henri IV**
**F-75181 Paris Cedex 04 (FR)**

(72) Inventeur : **Malfroot, Thierry**
**1 bis route de Tigery**
**F-91100 Saint-Germain-Les-Corbeil (FR)**
Inventeur : **Piteau, Marc**
**38 Avenue de Ballancourt**
**F-91710 Itteville (FR)**
Inventeur : **Senet, Jean-Pierre**
**79 rue de la Gare Herbeauvilliers-Buthiers**
**F-77760 La Chapelle-La-Reine (FR)**

**EP 0 045 234 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne le domaine de la synthèse chimique, plus précisément l'invention concerne un nouveau procédé de déalkylation des amines tertiaires par emploi de chloroformiates α-chlorés.

La question de la déalkylation des amines tertiaires est une question très importante dans le domaine de la synthèse chimique et en particulier dans le domaine de la synthèse pharmaceutique. En effet il existe de nombreux cas où l'on dispose d'une amine tertiaire dont on désire changer l'un des radicaux fixés à l'atome d'azote sans modifier le reste de la molécule. Le principe général de cette modification consiste à retirer le substituant non désiré de l'amine tertiaire par une réaction de déalkylation de manière à obtenir une amine secondaire selon le schéma réactionnel :

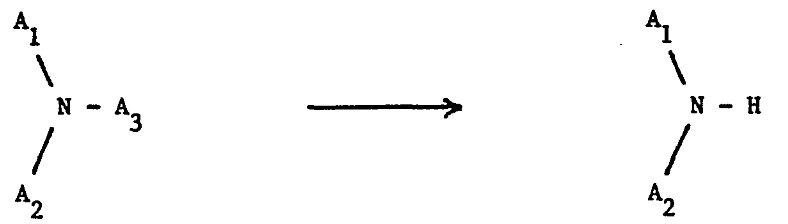
(1)

et à faire réagir l'amine secondaire ainsi obtenue avec un halogénure d'alkyle ou plus généralement avec un réactif d'alkylation de manière à obtenir l'amine tertiaire portant le substituant recherché selon le schéma réactionnel :

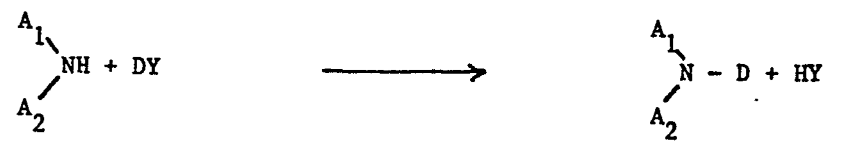
(2)

$A_1$, $A_2$, $A_3$, D représentant dans les schémas (1) et (2) un reste alkyle et Y représentant un accepteur d'hydrogène. Il en est ainsi en particulier dans le domaine de la synthèse pharmaceutique où l'on utilise de nombreuses amines tertiaires et notamment celles de la série morphinique. Les amines de base que l'on peut extraire des produits naturels nécessitent en règle générale au moins une substitution pour posséder l'activité optimale dans leur application ; cette substitution ne peut se faire qu'au travers d'une déalkylation préalable. Comme on peut le constater, la question de la déalkylation des amines tertiaires revêt une importance particulière dans le cadre de la synthèse chimique.

A l'heure actuelle il existe deux grandes voies pour effectuer la déalkylation des amines tertiaires.

La première de ces voies consiste à utiliser comme agents de déalkylation le bromure de cyanogène ou les chloroformiates d'éthyle et de benzyle.

Selon cette première voie qui est par exemple décrite dans les brevets américains 3 254 088, 3 493 657, 3 299 072 et 3 390 179, on fait réagir le bromure de cyanogène ou le chloroformiate sur l'amine à déalkyler et on traite le composé ainsi obtenu à l'acide chlorhydrique pour obtenir l'amine secondaire déalkylée. Cette première voie présente cependant un certain nombre d'inconvénients majeurs : elle manque de sélectivité vis-à-vis du radical à éliminer et ne permet pas de bons rendements, mais surtout elle fait appel à des conditions réactionnelles sévères et dangereuses notamment dans le cas du bromure de cyanogène.

La seconde de ces voies décrite dans les brevets américains 3 095 981 et 4 141 897 consiste à utiliser le chloroformiate de vinyle selon le schéma réactionnel suivant :

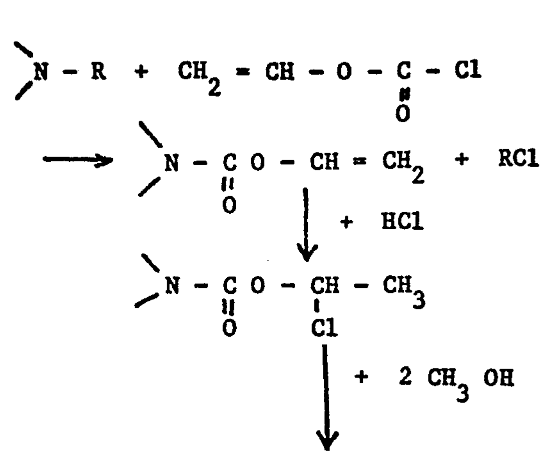

(3)
(4)
(5)

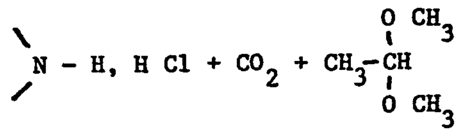

(6)

Ce procédé permet bien ainsi d'effectuer la déalkylation des amines tertiaires mais présente l'inconvénient de nécessiter un traitement à l'acide chlorhydrique. L'acide chlorhydrique introduit peut attaquer les autres substituants de l'atome d'azote et dégrader ou modifier la molécule, entraînant ainsi une baisse du rendement de l'opération de déalkylation et la nécessité de purifier l'amine déalkylée, opération qui est souvent très délicate.

La présente invention a précisément pour objet de proposer un procédé de déalkylation qui ne présente pas les inconvénients sus-mentionnés et notamment les risques de dégradation de l'amine par une attaque à l'acide chlorhydrique.

L'invention consiste donc en un procédé de déalkylation d'une amine tertiaire portant au moins un radical alkyle, de formule

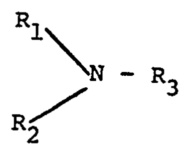

dans laquelle

$R_1$ et $R_2$ représentent des restes aliphatiques ou cycloaliphatiques, saturés ou non saturés, substitués ou non substitués, des restes aromatiques substitués ou non substitués, les radicaux $R_1$ et $R_2$ pouvant être chimiquement liés l'un à l'autre de manière à former un cycle substitué ou non substitué,

$R_3$ représente un reste aliphatique,

caractérisé en ce que l'on fait réagir sur l'amine

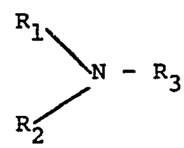

un chloroformiate α-chloré de formule

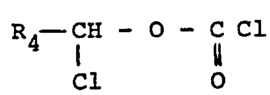

dans laquelle

$R_4$ représente un reste aliphatique saturé, substitué ou non substitué par des atomes d'halogènes, de manière à obtenir un carbamate α-chloré de formule

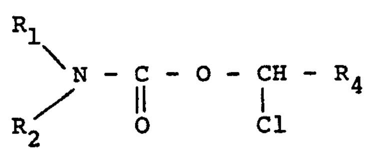

dans laquelle

$R_1$, $R_2$, $R_4$ ont la même signification que précédemment et à traiter le carbamate α-chloré ainsi obtenu avec un solvant hydroxylé léger de formule $R_5OH$ dans laquelle $R_5$ représente un radical aliphatique linéaire ou ramifié comportant de 1 à 4 atomes de carbone ou bien un atome d'hydrogène. Le traitement par le solvant hydroxylé léger provoque la coupure du carbamate α-chloré selon le schéma réactionnel suivant

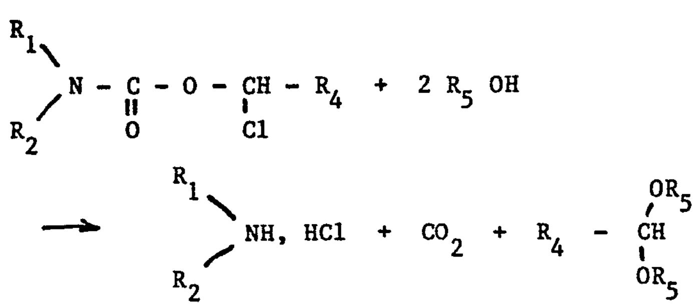

(7)

On récupère ainsi le chlorhydrate de l'amine déalkylée à partir duquel on peut aisément isoler l'amine déalkylée

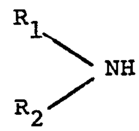

qui n'a subi aucune dégradation d'ordre chimique.

Le procédé selon l'invention permet ainsi, par l'emploi de chloroformiates α-chlorés, l'obtention directe, par une simple réaction de condensation, de carbamates α-chlorés à partir desquels il est aisé d'isoler le chlorhydrate de l'amine déalkylée, sans aucun risque de dégradation des autres substituants de l'amine puisqu'aucun traitement de l'acide chlorhydrique n'est nécessaire, contrairement au procédé décrit dans le brevet américain 3 905 981. Par ailleurs le procédé selon l'invention permet l'obtention directe, avec des rendements très bons, du chlorhydrate de l'amine déalkylée pure, ce qui rend possible l'utilisation ultérieure du chlorhydrate ou de l'amine sans nécessiter en général d'opération de purification poussée.

On donne ci-après une description détaillée de la mise en œuvre du procédé selon l'invention.

Comme il a été dit plus haut, le procédé selon l'invention consiste d'abord à faire réagir un chloroformiate α-chloré de formule

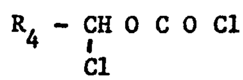

sur l'amine tertiaire

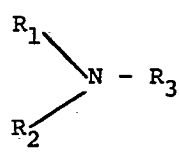

à déalkyler.

Le procédé selon l'invention s'applique à la plupart des amines tertiaires connues comportant au moins un radical aliphatique. Le reste de la molécule en dehors dudit radical aliphatique peut être constitué par deux radicaux distincts ou encore par un cycle aliphatique saturé ou insaturé, substitué ou non substitué comportant éventuellement au moins un hétéro-atome dans le cycle. Lorsque le reste de la molécule est constitué par deux radicaux distincts, ces radicaux, identiques ou différents, peuvent être des restes aliphatiques ou cycloaliphatiques, saturés ou non saturés, substitués ou non substitués, ou encore des restes aromatiques substitués ou non substitués. On peut ainsi citer à titre d'exemples comme amines tertiaires comportant au moins un radical aliphatique et susceptible d'être déalkylées par le procédé selon l'invention :

— des amines aliphatiques comme la triméthylamine, la triéthylamine,

— des amines cycloaliphatiques telles que la N-méthylpipéridine, la N-éthyl pipéridine, la tropine, la N-méthyl morpholine,

— des amines aromatiques comme la N,N diméthyl aniline, la N,N diéthyl aniline,

— des alcaloïdes comme la morphine, la codéine, l'α-cocaïne, la β-cocaïne, la thébaïne ou les N-alkyl acyloxy-14 morphinanes avec ou sans groupes alkoxy ou acyloxy en position 3 et avec ou sans substituants en position 6.

Ainsi, de préférence $R_1$ et $R_2$ comportent de 1 à 30 atomes de carbone s'ils sont distincts l'un de l'autre, et de 4 à 40 atomes de carbone s'ils sont reliés l'un à l'autre.

On fait donc réagir sur cette amine tertiaire un chloroformiate α-chloré de formule

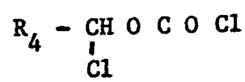

dans laquelle

$R_4$ représente un reste aliphatique saturé substitué ou non substitué par des atomes d'halogène. De tels chloroformiates α-chlorés ainsi que leur mode d'obtention sont notamment décrits dans la demande de brevet français 80 10606 au nom de la demanderesse. On préfèrera pour des questions de simplicité de mise en œuvre de l'invention et pour assurer les meilleurs rendements, les chloroformiates α-chlorés dans lesquels $R_4$ représente un reste alkyle inférieur et notamment le groupe méthyle, le groupe éthyle, le groupe propyle ou le groupe isopropyle. Le chloroformiate préféré selon l'invention est le chloroformiate de chloro-1 éthyle c'est-à-dire celui pour lequel le groupe $R_4$ est le groupe méthyle. Ce chloroformiate

s'obtient aisément en condensant en présence d'un catalyseur le phosgène sur l'acétaldéhyde selon le schéma réactionnel

$$CH_3CHO + COCl_2 \longrightarrow CH_3—CHClOCOCl \qquad (8)$$

La réaction de condensation du chloroformiate α-chloré sur l'amine tertiaire s'effectue à chaud et en général en présence d'un solvant et sous atmosphère anhydre. On utilise comme solvant un composé inerte vis-à-vis des réactifs introduits ; les hydrocarbures halogénés comme le dichloro-1,2 éthane ou le dichloro méthane conviennent bien en général. On peut également utiliser des solvants comme le tétrachlorure de carbone, le tétrahydrofuranne ou le toluène mais il faut signaler que ces solvants conduisent au moment de la condensation du chloroformiate α-chloré sur l'amine tertiaire à la formation de composés solides gênants, pour cette raison la demanderesse ne conseille pas l'emploi de ces solvants. Il est important que le solvant soit rigoureusement anhydre pour éviter toute décomposition du chloroformiate. La réaction s'effectue à chaud, sous reflux du solvant, il est donc conseillé d'utiliser un solvant ayant une température d'ébullition relativement élevée de manière à pouvoir opérer à température suffisamment élevée. Bien entendu il faut veiller à ce que la température de reflux du solvant choisi dans les conditions opératoires retenues reste inférieure aux températures de décomposition des réactifs ou du carbamate α-chloré en cours de formation. Dans ces conditions une durée de réaction de quelques heures est en général suffisante pour obtenir la transformation totale de l'amine en carbamate. Lorsque l'amine à déalkyler ne comporte qu'un seul radical alkyle c'est à la place de ce dernier que vient s'ajouter le chloroformiate α-chloré. Lorsque l'amine à déalkyler comporte plus d'un radical alkyle, le chloroformiate vient prendre la place du radical alkyle le plus apte à rompre sa liaison avec l'atome d'azote.

Le groupe $R_3$ de l'amine, qui est donc le groupe partant, est de préférence un radical aliphatique comportant jusqu'à 12 atomes de carbone et de préférence 1 à 8 atomes de carbone. On peut citer comme tels groupes les groupes méthyle, éthyle, propyle, butyle, benzyle, benzyle substitué, cyclohexyle et leurs homologues ramifiés éventuels. Compte tenu de l'excellente accessibilité des chloroformiates α-chlorés utilisés dans le cadre de la présente invention il est parfois avantageux, notamment lors des cas rebelles, d'employer un très fort excès dudit chloroformiate α-chloré par rapport à la stœchiométrie normale de la réaction, c'est-à-dire par rapport aux groupes à déalkyler. Cet excès peut aller jusqu'à remplacer le solvant inerte par le chloroformiate α-chloré lui-même. Il en résulte alors une séparation facilitée des produits réactionnels et, dans les cas où le rendement de la réaction en présence de solvants inerte est relativement bas, une très forte amélioration dudit rendement. Une température d'achèvement de la réaction comprise entre 35 et 150° permet de couvrir les différentes situations possibles, étant bien entendu que si l'amine à déalkyler est un produit très coûteux on préfère consommer du chloroformiate α-chloré (par décomposition thermique) plutôt que de renoncer à l'emploi d'une température élevée et à l'amélioration de quelques points du rendement. On apprécie, sur ce point que les chloroformiates α-chlorés conduisent par dégradation à des dérivés gem-dichlorés, tel que le dichloro-1,1 éthane et en $CO_2$ qui sont inoffensifs, alors que les chloroformiates vinyliques peuvent conduire à des résidus goudronneux (polymères mal définis) difficiles à éliminer.

Une fois que le chloroformiate α-chloré a totalement réagi sur l'amine, on laisse le mélange revenir à température ambiante et on ajoute la quantité nécessaire de solvant hydroxylé léger. Les alcanols permettent d'obtenir une coupure efficace du carbamate α-chloré sont les alcanols inférieurs comportant de 1 à 4 atomes de carbone. Les solvants hydroxylés légers préférés selon l'invention sont le méthanol, l'éthanol et l'eau qui permettent les meilleurs résultats. Le milieu réactionnel est alors agité et porté à une température légèrement supérieure à la température ambiante et préférentiellement comprise entre 35 et 40 °C. Après une durée d'agitation dans ces conditions comprise entre une demi-heure et une heure, la coupure du carbamate α-chloré par le solvant hydroxylé est totale. On élimine le solvant par distillation sous pression réduite et le chlorhydrate d'amine déalkylée est alors récupéré, lavé avec un hydrocarbure comme l'hexane et séché sous vide.

C'est ainsi l'un des avantages du procédé selon l'invention que de permettre l'obtention aisée de l'amine déalkylée sous forme de son chlorhydrate. Le procédé selon l'invention permet ainsi de traiter de nombreuses amines tertiaires et même des amines dans lesquelles l'atome d'azote est relié à un cycle aromatique comme les N,N dialkyl anilines pour lesquelles la déalkylation est réputée très difficile dans la littérature scientifique. La demanderesse a cependant observé que pour ces dernières amines les rendements sont très inférieurs à ceux obtenus généralement et qu'il faut de très longues durées de réaction pour obtenir la formation du carbamate α-chloré intermédiaire, à moins que, comme dit, on opère avec un excès de chloroformiate α-chloré, en guise de solvant. La mise en œuvre du procédé selon l'invention sera mieux comprise à l'aide des exemples donnés à titre non limitatif ci-après.

Exemple 1

De-éthylation de la N-éthyl pipéridine

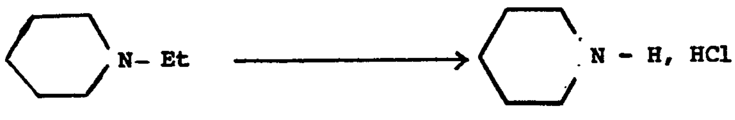

Mode opératoire

Dans un réacteur de 250 ml équipé d'un agitateur mécanique, d'un thermomètre, d'un réfrigérant ascendant et d'une ampoule de coulée, on introduit, après avoir purgé l'appareillage à l'azote, 10,12 g (0,070 8 mole) de chloroformiate de chloro-1-éthyle et 50 $cm^3$ de dichloro-1,2 éthane anhydre. On additionne ensuite, en maintenant la température entre − 5 et 0 °C, 8 g (0,070 8 mole) de N-éthyl pipéridine dissoute dans 10 $cm^3$ de dichloro-1,2 éthane anhydre. La durée de la coulée est d'environ 15 minutes. L'addition terminée, on porte le mélange réactionnel à la température de reflux du solvant (83 °C), l'agitation étant maintenue à cette opération pendant 1 heure. On laisse ensuite le mélange revenir à température ambiante et ajoute rapidement en une seule fois 40 $cm^3$ de méthanol.

Le milieu réactionnel est alors agité pendant 45 minutes à la température de 30-35 °C, puis le solvant est éliminé par évaporation sous pression réduite.

Le chlorhydrate de pipéridine obtenu est lavé à l'hexane et séché en dessicateur sous vide dynamique de 133 Pa (1 mm/Hg).

Obtenu : 8,5 g  Rendement : 98,8 %

Pt fusion : 246 °C  Littérature 244-245 °C

Spectre RMN : Pas d'impureté décelable en RMN.

Ce rendement est largement supérieur aux rendements rapportés à l'exemple 1 du brevet US 3 905 981 (avec les chloroformiates d'éthyle, de benzyle, de phényle et de vinyle).

Exemple 2

Déméthylation de la N-méthyl pipéridine

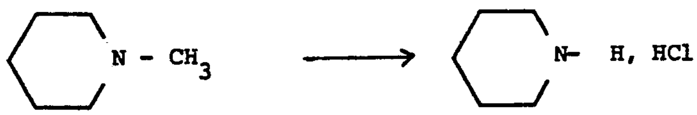

Dans un réacteur de 250 ml équipé d'un agitateur mécanique, d'un thermomètre, d'un réfrigérant ascendant et d'une ampoule de coulée, on introduit après avoir purgé l'appareillage à l'azote 10,01 g (0,07 mole) de chloroformiate d'α-chloro éthyle et 50 ml de dichloro-1,2 éthane anhydre.

On ajoute ensuite, en 15 min environ et en maintenant la température entre − 5 et 0 °C, 6, 93 g (0,07 mole) de N-méthyl pipéridine fraîchement distillée disssoute dans 10 ml de dichloro-1,2 éthane anhydre. Après l'addition de l'amine, on porte le mélange réactionnel à reflux sous agitation pendant 1 heure.

On laisse revenir à température ambiante et ajoute rapidement, en une seule fois, 40 ml de méthanol.

Le milieu réactionnel est alors agité pendant 45 min à la température de 35-40 °C puis le solvant est éliminé par évaporation sous pression réduite.

Le chlorhydrate de pipéridine obtenu est lavé au n-hexane et séché en dessicateur sous vide dynamique de 133 Pa (1 mm/Hg).

Obtenu : 8,3 g  Rendement : 97,6 %

Point de fusion 244 °C  Littérature : 244-245 °C

RMN : pas d'impureté décelable en RMN.

Ce rendement est également supérieur au rendement rapporté à l'exemple 2 de US 3 905 981 pour un produit qui nécessite encore un traitement d'élimination du groupe VOC.

Exemple 3

De-éthylation de la triéthylamine

$$Et_3N \rightarrow Et_2NH, HCl$$

L'essai a été réalisé avec l'appareillage et selon le mode opératoire décrit à l'exemple 1.

Quantités de matières utilisées :

— Chloroformiate d'α-chloro éthyle 10,01 g (0,07 mole) et 50 ml de dichloro-1,2 éthane

— Triéthylamine fraîchement distillée 7,07 g (0,07 mole) et 10 ml de dichloro-1,2 éthane.

On obtient 6,5 g de chlorhydrate de diéthylamine ce qui corresponnd à un rendement de 85 %.

Point de fusion : 229-230 °C

Littérature : 227-230 °C

Spectre RMN : pas d'impureté décelable en RMN.

Exemple 4

Débenzylation de la N,N diméthyl benzylamine

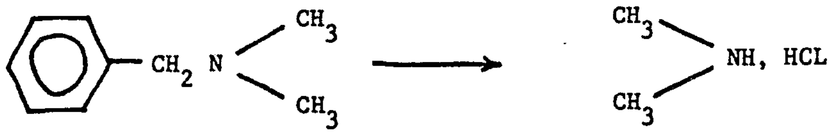

L'essai a été réalisé comme décrit à l'exemple 1 avec :

— 10,01 g (0,07 mole) de chloroformiate d'α-chloro éthyle et 50 ml de dichloro-1,2 éthane.

— 9,45 g (0,07 mole) de N,N diméthyl benzylamine fraîchement distillée et 10 ml de dichloro-1,2 éthane.

On obtient 5,7 g de chlorhydrate de diméthylamine ce qui correspond à un rendement de 91,5 % en produit pur.

Point de fusion : 165 °C

Littérature : 170-171 °C

Exemple 5

Déméthylation de la tropine, préparation du chlorhydrate de nortropine

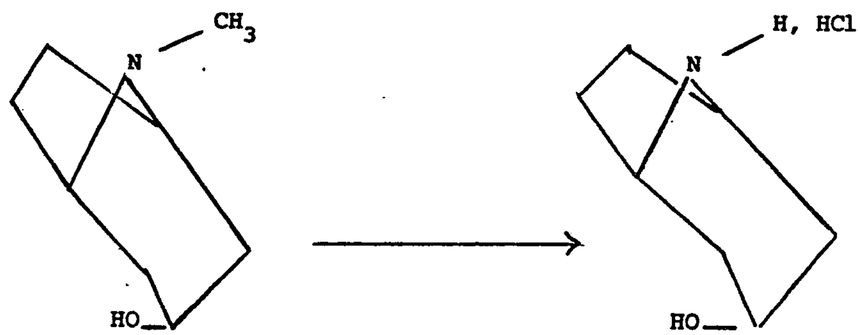

L'essai est réalisé comme décrit à l'exemple 1 avec :

— 20,0 g (0,14 mole) de chloroformiate d'α-chloro éthyle dans 50 ml de dichloro-1,2 éthane

— 6,5 g (0,046 mole) de tropine sublimée à 100 °C/39,9 Pa (0,3 mmHg) et 10 ml de dichloro-1,2 éthane.

On obtient 7,5 g de chlorhydrate de nortropine ce qui correspond à un rendement de 78 % en produit pur.

Point de fusion : 283 °C

Littérature : 285 °C

Exemple 6

Déméthylation de la N,N diméthylaniline

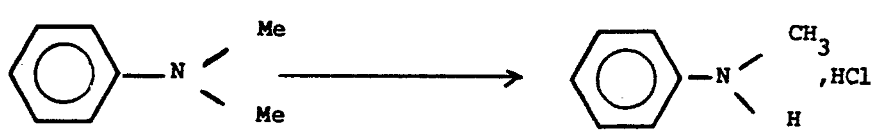

On utilise l'appareillage décrit à l'exemple 1.

On introduit dans le réacteur 10,1 g (0,07 mole) de chloroformiate d'α-chloroéthyle et 50 ml de dichloro-1,2 éthane anhydre. On ajoute ensuite en 15 min en maintenant la température entre – 5 et 0 °C, 8,47 g (0,07 mole) de diméthylaniline fraîchement distillée dans 10 ml de dichloro-1,2 éthane anhydre.

On porte à reflux pendant 30 h, refroidit à température ambiante, lave avec 2 fois 100 ml d'une solution aqueuse d'acide chlorhydrique diluée et une fois avec 100 ml d'eau.

On sèche la phase organique sur sulfate de magnésium et ajoute rapidement, en une seule fois, 40 ml de méthanol.

Le milieu réactionnel est alors agité pendant 1 h à la température de 35-40 °C puis le solvant est éliminé par évaporation sous pression réduite.

Le chlorhydrate de N-méthylaniline obtenu est lavé au n-hexane et séché en dessicateur sous vide dynamique de 133 Pa (1 mm/Hg).

Obtenu : 3,6 g Rendement : 36 % en produit pur
Point de fusion : 126 °C Littérature : 122-123 °C.
On obtient donc, en une seule étape réactionnelle, un produit extrêmement pur.

Exemple 7

Déethylation de la N-éthyl pipéridine par le chloroformiate de chloro-1 pentyle

Le mode opératoire étant identique à celui de l'exemple 1, les matières premières utilisées sont les suivantes :

— chloroformiate de chloro-1 pentyle : 7,4 g (0,04 mole) dans 30 ml de dichloro 1-2 éthane,
— N-éthyl pipéridine : 4,52 g (0,04 mole) dans 10 ml de dichloro 1-2 éthane

Après traitement au méthanol (25 ml) on obtient 4,6 g (94,5 %) de chlorhydrate de pipéridine fondant à 244 °C.

Exemple 8

Cet essai est identique à l'exemple 1 mais le méthanol est remplacé par la même quantité d'éthanol. On obtient 8,45 g (99,3 %) de chlorhydrate de pipéridine fondant à 244 °C.

Exemple 9

Cet essai est identique à l'exemple 1 mais on remplace le dichloro 1-2 éthane par du chlorure de méthylène. Deux heures de chauffage au reflux de ce solvant sont nécessaires pour réaliser la préparation du carbamate α-chloré.

A partir de 7,91 g (0,07 mole) de N-éthyl pipéridine on obtient 8,5 g (100 %) de chlorhydrate de pipéridine (pF : 244-245 °C).

Exemple 10

Cet essai est identique à l'exemple 9, le chlorure de méthylène étant remplacé par le tétrachlorure de carbone.

Le temps de reflux pour la préparation du carbamate α-chloré est de 1 heure. On obtient 8,3 g (97,6 %) de chlorhydrate de pipéridine fondant à 244-245 °C.

Exemple 11

Cet essai est identique à l'exemple 10 mais le solvant utilisé est le tétrahydrofuranne. On obtient 8,4 g (98,8 %) de chlorhydrate de pipéridine (pF : 244-245 °C).

Exemple 12

Cet essai est identique à l'exemple 10 mais le solvant utilisé est le toluène. On obtient 8,3 g (97,6 %) de chlorhydrate de pipéridine fondant à 244-245 °C.

Exemple 13

**Déméthylation de la N-méthyl morpholine**

Les conditions opératoires sont celles de l'exemple 1. En utilisant 7,07 g (0,07 mole) de N-méthyl morpholine et 10,01 g (0,07 mole) de chloroformiate d'α-chloro éthyle on obtient 8,3 g (96 %) de chlorhydrate de morpholine fondant à 174-177 °C (litt : 175 °C).

Exemple 14 à 17

Comme l'exemple 6 précédent le montre, l'invention permet de déalkyler les amines aromatiques en dépit de la difficulté de cette entreprise. Dans la série d'expériences suivantes on a montré l'intérêt de procéder avec du chloroformiate α-chloré pour tout solvant. La première expérience a consisté à tenter la monodéethylation du diéthylamino-8 tétrahydro-1, 2, 3, 4 dibenzofuranne (DTB), à l'aide de chloroformate de vinyle.

On s'est placé dans les conditions de l'exemple 1, de US 3 905 981, c'est-à-dire dans le dichloro-1, 2, éthane, en remplaçant la N-éthyl pipéridine par la même quantité en moles de DTB.

Aucune réaction ne se produit même si l'on prolonge à 48 heures la durée du chauffage à reflux.

On s'est placé (ex. 15) dans les conditions précédentes mais on a remplacé le chloroformiate de vinyle par du chloroformiate d'α-chloroéthyle et on a pratiqué l'introduction de l'amine à − 5 °C au lieu de − 35 °C.

L'évolution de la réaction a été suivie lors du chauffage à reflux, par chromatographie en phase vapeur (SE 30, colonne de 3,2 mm de diamètre, longueur 2,5 mètres, à 250 °C, avec une pression d'entrée de 215 820 Pa (2,2 kg/cm$^2$). Les ponctions ayant été traitées à la soude, on a constaté que la réaction est lente.

Au bout de 48 heures on a obtenu un rendement de 40 % en amine monodéethylée, en soumettant le mélange réactionnel au traitement suivant : décomposition par MeOH en excès en une heure à 40 °C, puis coulage dans une solution aqueuse de soude à 10 %, extraction à l'éther, acidification de la phase organique par HCl à 10 %, lavage de la phase aqueuse à l'éther, retour en milieu basique et réextraction à l'éther.

Dans une nouvelle expérience (ex. 16) on a opéré sans solvant. On a placé dans un réacteur de 50 ml 2 mM de DTB (soit 480 mg) et 3 ml (3,7 g soit 26 mM) de chloroformiate d'α-chloroéthyle. On a chauffé à 130 °C et laissé la réaction se poursuivre pendant 30 heures. Le mélange réactionnel a alors été soumis au même traitement qu'à l'exemple 15 et on a obtenu un rendement nettement amélioré, égal à 83 %.

On a réitéré (ex. 17) la réaction précédente à partir de 5,6 mM de DTB et 11,2 mM de chloroformiate α-chloré. On a obtenu, toujours au bout de 30 heures à 130 °C, 89 % de produit isolé de la même manière.

Exemple 18

On a reproduit l'exemple 16 à l'identique, en remplaçant seulement le DTB par la diamine de formule

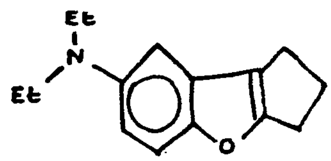

qui ne diffère du DTB que par un atome de carbone de moins dans le cycle cycloaliphatique latéral.

On a obtenu un rendement de 80 % en produit monodéethylé isolé.

Exemple 19

On a reproduit l'exemple 16 en remplaçant le DTB par le diméthylamino-8 tétrahydro-1,2,3,4 benzofuranne et en modifiant le temps de réaction.

Au bout d'une heure seulement à 130 °C on a obtenu un rendement de 86 % en produit monodéméthylé isolé.

Exemple 20

Dans un radiateur de 250 cm$^3$ équipé d'un agitateur mécanique, d'un thermomètre, d'une ampoule de coulée et d'un réfrigérant ascendant, on introduit 10,12 g (0,078 mole) de chloroformiate de chloro 1 éthyle et 50 cm$^3$ de dichloro 1-2 éthane anhydre. On additionne ensuite 8 g de N-éthyl pipéridine (0,070 8 mole) en solution dans 10 cm$^3$ de dichloro 1-2 éthane anhydre en maintenant la température entre − 5 et 0 °C.

La coulée terminée, le mélange réactionnel est porté 1 heure au reflux du solvant (83 °C), puis ramené à température ambiante, le solvant est évaporé à l'évaporateur relatif. Au résidu obtenu on ajoute 40 cm$^3$ de THF et 2 cm$^3$ d'eau, ce mélange est agité 1 h 1/2 à 40 °C — (Analyse IR → $\nu$C = 0 à 1 720 cm$^{-1}$ signifie qu'il reste du carbonate). On rajoute au mélange précédent 2,5 cm$^3$ d'eau et l'on agite de nouveau 1 h 1/2 au reflux du THF (66 °C) (analyse IR → il reste toujours le carbamate).

Après ces 3 heures de traitement, le taux de transformation du carbamate en chlorhydrate n'étant que de 50 % environ, nous avons rajouté 40 cm$^3$ de méthanol et agité 45 minutes à 40-45 °C. On a pu alors recueillir le chlorhydrate de pipéridine avec un bon rendement. On voit que l'eau permet de passer du carbamate au chlorhydrate d'amine secondaire mais que le méthanol lui est supérieur dans cette fonction.

Toutefois on obtient de meilleurs résultats lorsqu'on utilise l'eau si, dans le mode opératoire précédent, après évaporation du dichloro-1,2 éthane, on n'ajoute que de l'eau (10 cm$^3$) et qu'on chauffe entre 60 et 80 °C pendant 1 à 3 heures.

**Revendications**

1. Procédé de déalkylation d'une amine tertiaire, portant au moins un radical alkyle, de formule

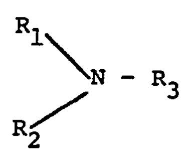

dans laquelle

$R_1$ et $R_2$ représentent des restes aliphatiques ou cycloaliphatiques, saturés ou non saturés, substitués ou non substitués, des restes aromatiques substitués ou non substitués, les radicaux $R_1$ et $R_2$ pouvant être chimiquement liés l'un à l'autre de manière à former un cycle substitué ou non substitué,

$R_3$ représente un reste aliphatique,

caractérisé en ce que l'on fait réagir sur l'amine

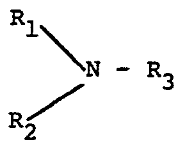

un chloroformiate α-chloré de formule

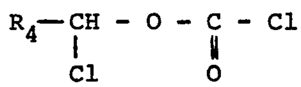

dans laquelle

$R_4$ représente un reste aliphatique saturé substitué ou non substitué par des atomes d'halogène, de manière à obtenir un carbamate α-chloré de formule

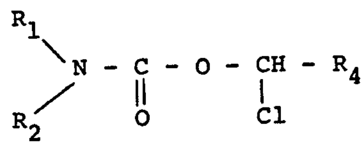

dans laquelle

$R_1$, $R_2$, $R_4$ ont la même signification que précédemment, et on traite le carbamate α-chloré ainsi obtenu avec un solvant hydroxylé léger de formule $R_5OH$ dans laquelle $R_5$ représente un radical aliphatique linéaire ou ramifié comportant de 1 à 4 atomes de carbone ou un atome d'hydrogène, de manière à obtenir l'amine déalkylée de formule

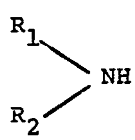

dans laquelle

$R_1$ et $R_2$ ont la signification précédente.

2. Procédé selon la revendication 1, caractérisé en ce que le radical $R_4$ dudit chloroformiate α-chloré est un reste alkyle inférieur.

3. Procédé selon la revendication 2, caractérisé en ce que ledit chloroformiate α-chloré est le chloroformiate de chloro-1 éthyle.

4. Procédé selon l'une quelconque des revendications 1 à 3, caractérisé en ce que ledit solvant hydroxylé léger est le méthanol.

5. Procédé selon l'une quelconque des revendications 1 à 4, caractérisé en ce que $R_3$ est un radical aliphatique comportant de 1 à 12 atomes de carbone.

6. Procédé selon l'une quelconque des revendications 1 à 5, caractérisé en ce que ladite amine est choisie dans le groupe constitué par la triméthyl amine, la triéthyl amine, la N-méthyl pipéridine, la N-éthyl pipéridine, la tropine, la N-méthyl morpholine, la N,N-diméthylaniline, la N,N diéthyl aniline, la morphine, la codéine, l'α-cocaïne, la β-cocaïne, la thébaïne et les N-alkyl acyloxy-14 morphinanes.

**7. Procédé selon l'une quelconque des revendications 1 à 6, caractérisé en ce qu'on effectue la réaction de l'amine sur le chloroformiate α-chloré en présence d'un solvant hydrocarbure halogéné.**

8. Procédé selon l'une quelconque des revendications 1 à 7, caractérisé en ce qu'on effectue la réaction de l'amine sur le chloroformiate α-chloré en l'absence de solvant, en présence d'un excès de chloroformiate α-chloré.

**Claims**

1. Process for the dealkylation of a tertiary amine carrying at least one alkyl radical of the formula

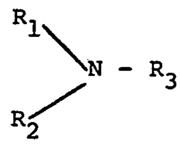

in which

$R_1$ and $R_2$ represent saturated or unsaturated, substituted or unsubstituted aliphatic or cycloaliphatic radicals or substituted or unsubstituted aromatic radicals, it being for the radicals $R_1$ and $R_2$ to be chemically bonded to one another so as to form a substituted or unsubstituted ring, and

$R_3$ represents an aliphatic radical,

characterised in that an α-chlorinated chloroformate of the formula

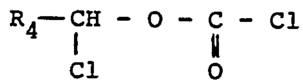

in which

$R_4$ represents a saturated aliphatic radical which is unsubstituted or substituted by halogen atoms, is reacted with the amine

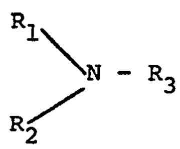

so as to obtain an α-chlorinated carbamate of the formula

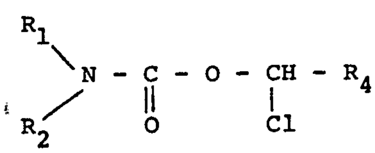

in which

$R_1$, $R_2$ and $R_4$ have the same meaning as above, and in that the α-chlorinated carbamate thus obtained is treated with a light hydroxylated solvent of the formula $R_5$, in which $R_5$ represents a linear or branched aliphatic radical containing from 1 to 4 carbon atoms or a hydrogen atom, so as to obtain the dealkylated amine of the formula

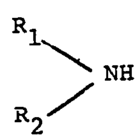

in which

$R_1$ and $R_2$ have the above meanings.

2. Process according to Claim 1, characterised in that the radical $R_4$ of the said α-chlorinated chloroformate is a lower alkyl radical.

3. Process according to Claim 2, characterised in that the said α-chlorinated chloroformate is 1-chloroethyl chloroformate.

4. Process according to any one of Claims 1 to 3, characterised in that the said light hydroxylated solvent is methanol.

5. Process according to any one of Claims 1 to 4, characterised in that $R_3$ is an aliphatic radical containing from 1 to 12 carbon atoms.

6. Process according to any one of Claims 1 to 5, characterised in that the said amine is chosen from the group comprising trimethylamine, triethylamine, N-methylpiperidine, N-ethylpiperidine, tropine, N-methylmorpholine, N,N-dimethylaniline, N,N-diethylaniline, morphine, codeine, α-cocaine, β-cocaine, thebaine and N-alkyl 14-acyloxy morphinans.

7. Process according to any one of Claims 1 to 6, characterised in that the reaction of the amine with the α-chlorinated chloroformate is carried out in the presence of a halogenated hydrocarbon solvent.

8. Process according to any one of Claims 1 to 7, characterised in that the reaction of the amine with the -chlorinated chloroformate is carried out in the absence of a solvent, in the presence of an excess of α-chlorinated chloroformate.

**Ansprüche**

1. Verfahren zur Entalkylierung eines tertiären Amins mit mindestens einem Alkylrest der Formel

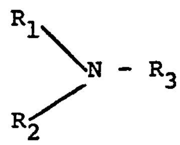

in der

$R_1$ und $R_2$ aliphatische oder cycloaliphatische Reste sind, die gesättigt oder ungesättigt, substituiert oder unsubstituiert sind, oder substituierte oder unsubstituierte aromatische Reste bedeuten, wobei $R_1$ und $R_2$ chemisch zu einem substituierten oder unsubstituierten Ring verbunden sein können,

$R_3$ ein aliphatischer Rest ist,

dadurch gekennzeichnet, daß man ein Amin der Formel

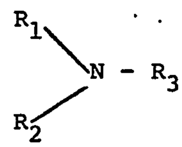

mit einem α-chlorierten Chlorformiat der Formel

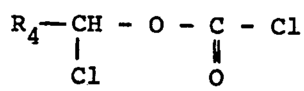

in der

$R_4$ ein gesättigter aliphatischer Rest ist, der mit Halogenatomen substituiert oder unsubstituiert ist, zu einem α-chlorierten Carbamat der Formel

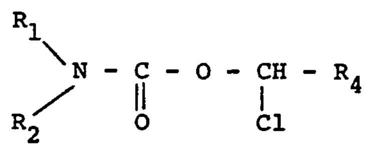

umsetzt, in der

$R_1$, $R_2$ und $R_4$ die oben angegebene Bedeutung haben, das so erhaltenen α-chlorierte Carbamat mit einem leichten, Hydroxylgruppen enthaltenden Lösungsmittel der Formel $R_5OH$ behandelt, in der $R_5$ ein **geradkettiger oder verzweigter aliphatischer Rest mit 1 bis 4 Kohlenstoffatomen oder ein Wasserstoffatom ist, und auf diese Weise das entalkylierte Amin der Formel**

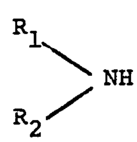

erhält, in der

$R_1$ und $R_2$ **oben angegebene Bedeutung haben.**

2. Verfahren nach Anspruch 1, dadurch gekennzeichnet, daß der Rest $R_4$ des genannten α-chlorierten Chlorformiats ein niederer Alkylrest ist.

3. Verfahren nach Anspruch 2, dadurch gekennzeichnet, daß das genannte α-chlorierte Chlorformiat das 1-Chlorethyl-chlorformiat ist.

4. Verfahren nach einem der Ansprüche 1 bis 3, dadurch gekennzeichnet, daß das genannte leichte, Hydroxylgruppen enthaltende Lösungsmittel Methanol ist.

5. Verfahren nach einem der Ansprüche 1 bis 4, dadurch gekennzeichnet, daß $R_3$ ein aliphatischer Rest mit 1 bis 12 Kohlenstoffatomen ist.

6. Verfahren nach einem der Ansprüche 1 bis 5, dadurch gekennzeichnet, daß das genannte Amin aus der aus Trimethylamin, Triethyıamin, N-Methylpiperidin, N-Ethylpiperidin, Tropin, N-Methylmorpholin, N,N-Dimethylanilin, N,N-Diethylanilin, Morphin, Codein, α-Cocain, Thebain und N-Alkylacyloxy-14-morphinanen bestehenden Gruppe ausgewählt wird.

7. Verfahren nach einem der Ansprüche 1 bis 6, dadurch gekennzeichnet, daß man die Reaktion des Amins mit dem α-chlorierten Chlorformiat in Gegenwart eines halogenierten Kohlenwasserstoffs als Lösungsmittel durchführt.

8. Verfahren nach einem der Ansprüche 1 bis 7, dadurch gekennzeichnet, daß man die Reaktion des Amins mit dem α-chlorierten Chlorformiat in Abwesenheit des Lösungsmittel in Gegenwart eines Überschusses an α-chloriertem Chlorformiat durchführt.